# EUROPEAN PATENT SPECIFICATION

(11) **EP 3 498 264 B1**
(45) Date of publication and mention of the grant of the patent: **08.11.2023**
(21) Application number: 17839879.8
(22) Date of filing: 11.08.2017
(51) Int. Cl.: A61K 9/16, A61K 9/50, A61K 47/38, A61K 47/32, A61K 45/06, A61K 31/18, A61K 31/58, A61K 31/4709, A61K 31/4725, A61K 31/4985, A61P 13/08, A61P 43/00

(54) **PHARMACEUTICAL PREPARATION FOR ORAL ADMINISTRATION WITH CONTROLLED DISSOLUTION RATE, THE PREPARATION COMPRISING TAMSULOSIN HYDROCHLORIDE-CONTAINING SUSTAINED-RELEASE PELLETS**
PHARMAZEUTISCHE ZUBEREITUNG MIT TAMSULOSINHYDROCHLORIDHALTIGEN PELLETS MIT VERZÖGERTER FREISETZUNG ZUR ORALEN VERABREICHUNG MIT KONTROLLIERTER AUFLÖSUNGSGESCHWINDIGKEIT
PRÉPARATION PHARMACEUTIQUE POUR ADMINISTRATION ORALE À VITESSE DE DISSOLUTION CONTRÔLÉE, LA PRÉPARATION COMPRENANT DES PASTILLES À LIBÉRATION PROLONGÉE CONTENANT DU CHLORHYDRATE DE TAMSULOSINE

(30) Priority: 12.08.2016 KR 20160103269
(43) Date of publication of application: 19.06.2019
(73) Proprietor: Hanmi Pharm. Co., Ltd., Hwaseong-si, Gyeonggi-do 18536 (KR)
(72) Inventor: KIM, Jae Ho, Suwon-si Gyeonggi-do 16336 (KR); LEE, Benjamin Joon, Hwaseong-si Gyeonggi-do 18315 (KR); KIM, Jin Cheul, Seoul 07216 (KR); KIM, Yong Il, Suwon-si Gyeonggi-do 16325 (KR); PARK, Jae Hyun, Suwon-si Gyeonggi-do 16707 (KR); WOO, Jong Soo, Seoul 05555 (KR)
(74) Representative: Marks & Clerk LLP
(86) International application number: PCT/KR2017/008800
(87) International publication number: WO 2018/030862

(56) References cited:
- WO-A2-2016/133333
- KR-A- 20050 031 432
- KR-A- 20060 093 931
- KR-A- 20070 094 110
- KR-A- 20110 102 339
- KR-A- 20110 113 940
- Anonymous: "Flomax (tamsulosin hydrochloride) Capsules, 0.4 mg Prescribing Information", Drugs@FDA Instructions: Regulatory Information, 5 October 2005 (2005-10-05), pages 1-20, XP055664689, Retrieved from the Internet: URL:https://www.accessdata.fda.gov/drugsat fda_docs/label/2005/020579s016lbl.pdf [retrieved on 2020-02-04]

## Description

### TECHNICAL FIELD

The present disclosure relates to a pharmaceutical formulation for oral administration, the formulation including tamsulosin hydrochloride-containing sustained-release pellets, and more particularly, a pharmaceutical formulation for oral administration, the formulation including tamsulosin hydrochloride-containing sustained-release pellets, wherein the sustained-release pellets enable precise release of tamsulosin hydrochloride while passing through the gastrointestinal tract.

### BACKGROUND ART

Tamsulosin is a compound having a structure of the following Chemical Formula 1 and a general name of (R)-5-[2-[[2-(2-ethoxyphenoxy)ethyl]-amino]propyl]-2-methoxy-benzenesulfonamide.

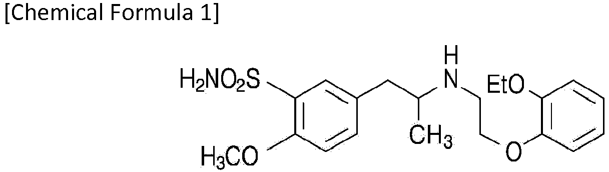

Tamsulosin or pharmaceutically acceptable salts thereof are known to have α-sympathetic nerve-blocking activity that is useful in the treatment of heart failure and benign prostatic hyperplasia. Among these, tamsulosin hydrochloride selectively inhibits α-adrenoceptors to selectively act on the urogenital organ, and thus relaxes smooth muscle surrounding the bladder and the prostate, resulting in the improvement of urinary flow rates. Therefore, tamsulosin hydrochloride is known to have excellent pharmacological effects which ameliorate symptoms of benign prostatic hyperplasia (BPH) while having few side effects.

Tamsulosin hydrochloride is well absorbed with bioavailability of 90% or more, and has a long half-life of approximately 9 to 13 hours in healthy subjects and 14 to 15 hours in BPH patients. Therefore, when a sustained release formulation of tamsulosin hydrochloride is prepared to slowly release tamsulosin hydrochloride for about 6 hours, the concentration of the drug may be sufficiently maintained for 24 hours.

Tamsulosin hydrochloride is a white crystalline powder having physiochemical properties such as decomposition at approximately 230 °C and slight solubility in water. Since tamsulosin hydrochloride is not a substance that is difficult to dissolve in water, it is necessary to precisely control its release for a sustained-release formulation.

Patent Document 1 discloses a method of preparing a tamsulosin formulation for the development of a tamsulosin sustained-release formulation, the method including preparing granules containing an enteric release controlling agent and filling a capsule with the granules. However, when only the enteric release controlling agent is used as described above, release of the active ingredient into serous fluid is not properly controlled, and it is difficult to achieve uniform dissolution patterns of the active ingredient according to pH changes in the gastrointestinal tract.

Patent Document 2 discloses a capsule formulation including tamsulosin hydrochloride sustained-release granules containing tamsulosin hydrochloride, polyvinyl acetate, hydroxypropyl methylcellulose, and a granule-forming material. Since this formulation uses polyvinyl acetate and hydroxypropyl methylcellulose, the active ingredient is able to be released over time after administration, regardless of pH changes or the presence or absence of foods. However, because the active ingredient is released in a time-dependent manner, there is a disadvantage in that the drug is discharged out of the body before complete release of the active ingredient or the release may not be precisely controlled at a target time point and site.

Further, the tamsulosin hydrochloride formulation of Patent Document 2 and commercially available formulations all have problems in that a large batch-to-batch variation in the pellet size exists during preparation, and thus the dissolution rate is not constant.

Patent Document 3 discloses a controlled release formulation which comprises a weak basic drug which is an active ingredient, shows pH dependent solubility and is selected from the group consisting of doxazocin, tamsulosin, paroxetine, tolterodine, venlafaxin, and a pharmaceutically acceptable salt thereof. The formulation comprises a matrix layer containing the active ingredient and includes a swelling hydrophilic polymer, a pH dependent polymer and other excipients.

Furthermore, commercially available tamsulosin hydrochloride formulations including Flomax^{®} (made by Boehringer Ingelheim) are all capsules for oral administration, in which 0.4 mg of tamsulosin hydrochloride is contained in a plurality of pellets. The total weight of the pellets containing 0.4 mg of tamsulosin hydrochloride is about 330 mg. Therefore, when the formulation is used as a composite formulation, the size of the composite formulation increases. Considering that prostatic hyperplasia is more common in older men, it is not easy to swallow, and thus there is concern about poor drug compliance.

### [Prior Art Documents]

### [Patent Documents]

Korean Patent Publication No. 1993-0007245 B1
Korean Patent Publication No. 2005-0082038 A
Korean Patent Publication KR 2007 0094110 A

### DESCRIPTION OF EMBODIMENTS

### TECHNICAL PROBLEM

The present invention provides a pharmaceutical formulation for oral administration comprising tamsulosin hydrochloride-containing sustained-release pellets, each pellet comprising (i) tamsulosin hydrochloride, (ii) hydroxypropyl methylcellulose (HPMC), (iii) an acid-resistant acrylic polymer, and (iv) two or more kinds of insoluble diluent, wherein the pharmaceutical formulation for oral administration is prepared by a method comprising: (a) mixing and extruding a mixture comprising (i) tamsulosin hydrochloride, (ii) hydroxypropyl methylcellulose (HPMC), (iii) an acid-resistant acrylic polymer, and (iv) two or more kinds of insoluble diluent to mold the mixture in the form of granules; and (b) spheronizing the extruded granules using a spheronizer at a rotation speed of about 600 rpm to about 800 rpm for about 10 minutes to about 45 minutes to prepare pellets, wherein the sustained-release pellets each comprise (i) 0.07% by weight to 0.50% by weight of tamsulosin hydrochloride, (ii) 1.0% by weight to 7.0% by weight of hydroxypropyl methylcellulose (HPMC), (iii) 3.0% by weight to 20.0% by weight of an acid-resistant acrylic polymer, and (iv) 70.0% by weight to 95.9% by weight of two or more kinds of insoluble diluent, based on a dry pellet, and wherein the hydroxypropyl methylcellulose (HPMC) has a viscosity of 10000 cPs to 100000 cPs.

Another aspect of the present disclosure is to provide a method of preparing the pharmaceutical formulation for oral administration, the formulation including the tamsulosin hydrochloride-containing sustained-release pellets.

Still another aspect of the present disclosure is to provide a tamsulosin-containing hard capsule composite formulation including the tamsulosin-containing sustained-release pellets together with a pharmaceutical composition of one or more of different therapeutic classes, each being physically separated from the pellets.

### SOLUTION TO PROBLEM

An aspect of the present disclosure is to provide a pharmaceutical formulation for oral administration, the formulation including tamsulosin hydrochloride-containing sustained-release pellets, wherein (i) tamsulosin hydrochloride, (ii) hydroxypropyl methylcellulose (HPMC), (iii) an acid-resistant acrylic polymer, and (iv) two or more kinds of insoluble diluent are included.

Another aspect of the present disclosure is to provide a method of preparing the pharmaceutical formulation for oral administration according to an aspect of the present disclosure, the method including (a) mixing and extruding a mixture containing (i) tamsulosin hydrochloride, (ii) hydroxypropyl methylcellulose (HPMC), (iii) an acid-resistant acrylic polymer, and (iv) two or more kinds of insoluble diluent to mold the mixture in the form of granules; and (b) spheronizing the extruded granules using a spheronizer at a rotation speed of about 600 rpm to about 800 rpm for about 10 minutes to about 45 minutes to prepare the pellets.

Still another aspect of the present disclosure is to provide a hard capsule composite formulation containing tamsulosin hydrochloride comprising (A) tamsulosin hydrochloride-containing sustained-release pellets, each pellet containing (i) tamsulosin hydrochloride, (ii) hydroxypropyl methylcellulose(HPMC), (iii) an acid-resistant acrylic polymer, and (iv) two or more kinds of insoluble diluent; and (B) a pharmaceutical composition physically separated from the tamsulosin hydrochloride-containing sustained-release pellets, the pharmaceutical composition containing one or more different pharmaceutically active ingredients and a pharmaceutically acceptable carrier.

### ADVANTAGEOUS EFFECTS OF DISCLOSURE

A pharmaceutical formulation for oral administration according to an aspect of the present disclosure has a small batch-to-batch variation in the pellet size during preparation, as compared with known tamsulosin hydrochloride formulations, and thus production reproducibility is very excellent. Due to the small variation in the pellet size, the pharmaceutical formulation exhibits a constant dissolution rate and a small variation in the dissolution rate. Such release behavior is expected to work equally in a patient's body and is expected to exhibit an excellent effect in the treatment of the patient.

Further, in the present disclosure, a pH-dependent enteric base material may be used together with a non-pH-dependent enteric base material to more precisely control the dissolution rate in the body. The weight of the tamsulosin hydrochloride-containing pellet may be greatly reduced by 1/2 of those of commercially available products, and thus the pellets may be used in a much smaller amount than those of the known products, when an equal dose of tamsulosin hydrochloride is used. As described above, the tamsulosin hydrochloride-containing sustained-release pellet according to the present disclosure may include the same dose of tamsulosin hydrochloride, even though used in a smaller amount. Accordingly, when the pellet is used in the preparation of a composite formulation including tamsulosin hydrochloride and one or more different pharmaceutically active ingredients, the formulation may be provided in a much smaller size than known formulations while containing the same dose of the active ingredient per a final unit formulation, and thus there is an advantage in that a patient's drug compliance may be improved.

### BRIEF DESCRIPTION OF DRAWINGS

FIG. 1 shows a photograph of tamsulosin hydrochloride-containing sustained-release pellets according to one exemplary embodiment of the present disclosure;
FIGS. 2A to 2C show results of measuring dissolution rates of dry pellets prepared in Examples 1 to 5, and 13 and Comparative Examples 1, 3 to 5, 7, 8, and 10 of the present disclosure;
FIG. 3 shows a graph of particle sizes of pellets prepared according to Examples 1 to 10 of the present disclosure;
FIGS. 4A to 4C show graphs of dissolution rates of three different batches, which were measured during a dissolution test for 1 hour in a phosphate buffer at pH 6.8, to examine reproducibility of tamsulosin hydrochloride capsule formulations prepared in Examples and Comparative Examples of the present disclosure according to production batches; and
FIGS. 5A to 5D show illustrations of hard capsule composite formulations, each including tamsulosin-containing sustained-release pellets according to one specific embodiment of the present disclosure together with a different pharmaceutically active ingredient.

### BEST MODE

Unless defined otherwise, all technical terms used herein have the same meanings as those generally understood by one of ordinary skill in the art to which the present disclosure belongs.

The present inventors have conducted intensive studies on a pharmaceutical formulation for oral administration including tamsulosin hydrochloride-containing sustained-release pellets, wherein a batch-to-batch variation in the pellet size is small during preparation, and thus dissolution rates are maintained constant. As a result, it was found that when hydroxypropyl methylcellulose and an acid-resistant acrylic polymer are used in combination at a predetermined weight ratio with respect to the active ingredient during preparation of sustained-release pellets, the finally prepared sustained-release pellets are similar to each other in the size, and a variation in the pellet size is small, and thus dissolution rates of tamsulosin hydrochloride between batches are maintained constant, and a volume of the finally prepared tamsulosin hydrochloride-containing sustained-release pellet may be reduced to prepare a unit formulation of a small size, thereby completing the present disclosure.

An aspect of the present disclosure provides a pharmaceutical formulation for oral administration, the formulation including tamsulosin hydrochloride-containing sustained-release pellets containing (i) tamsulosin hydrochloride, (ii) hydroxypropyl methylcellulose (HPMC) which is a non-pH-dependent polymer base material, (iii) an acid-resistant acrylic polymer which is a pH-dependent polymer base material, and (iv) two or more kinds of insoluble diluent.

The sustained-release pellets included in the pharmaceutical formulation for oral administration each may include tamsulosin hydrochloride which is a pharmaceutically active ingredient in an amount of about 0.07% by weight to 0.50% by weight, specifically about 0.15% by weight to 0.40% by weight, more specifically about 0.20% by weight to 0.35% by weight, based on a dry pellet.

The hydroxypropyl methylcellulose (HPMC, hypromellose) is a non-pH-dependent polymer, and plays a role in controlling a dissolution rate of tamsulosin hydrochloride from the sustained-release granule formulation, regardless of pH. In other words, when hydroxypropyl methylcellulose, together with other ingredients, are dissolved in an aqueous solution, initial release of the active ingredient through formed pores is controlled, and therefore, the active ingredient is continuously slowly released. A viscosity of the hydroxypropyl methylcellulose may be 10,000 cPs or more, and in a specific embodiment, the viscosity may be 10,000 cPs to 100,000 cPs, and more specifically, 15,000 cPs to 100,000 cPs (100,000 cPs = 100 Pa.s).

The hydroxypropyl methylcellulose having the viscosity in the above range may be, for example, METOLOSE 60SH, 65SH, 90SH (Shin-Etsu, Japan), etc. When the viscosity of hydroxypropyl methylcellulose is lower than the above range, sustained release of the active ingredient of the sustained-release granule may be difficult even though the hydroxypropyl methylcellulose is used in a larger amount during preparation of the sustained-release granule. The sustained-release pellet may include high-viscosity hypromellose (hydroxypropyl methylcellulose, 10,000 cps to 100,000 cps) in an amount of about 1.0% by weight to 7.0% by weight, specifically about 1.0% by weight to 5.0% by weight, more specifically about 1.5% by weight to 4.0% by weight, based on the dry pellet. When the hydroxypropyl methylcellulose is used in a larger amount than the above range, the sustained-release capability becomes excessive, and thus release of the drug is too retarded. In addition, the granules stick to each other during the production to generate a problem of productivity reduction. When the amount is smaller than the above range, there is a significant variation in the pellet size to increase a ratio of fine powder, and as a result, rapid release of the drug occurs and it is difficult to obtain a sustained-release capability in a desired level.

The acid-resistant acrylic polymer is a pH-dependent enteric polymer that does not dissolve under strong acidic conditions (about pH 4.5 or lower) and dissolves at pH 5.5 or higher, and may be selected from the group consisting of copolymers of methacrylic acid and methyl methacrylate, for example, Eudragit L30 D-55, Eudragit L100, Eudragit L100 D-55, Eudragit L12,5, Eudragit S100, Eudragit S12,5, Eudragit FS 30 D, and any combination thereof. In a specific embodiment, the acid-resistant acrylic polymer may be selected from the group consisting of Eudragit L30 D-55, Eudragit L100, D-55, Eudragit L12,5, and combinations thereof. Eudragit L30 D-55 hardly dissolves at a low pH (e.g., pH 5.5 or lower), but start to dissolve under condition of pH 5.5 or higher. Due to this characteristic, Eudragit L30 D-55 is an excipient widely used as an enteric base material. Eudragit L30 D-55 is available as a 30% (m/V) aqueous dispersion of an acrylate polymer containing sodium lauryl sulfate and polysorbate 80 as emulsifiers.

The sustained-release pellet of the present disclosure may include the acid-resistant acrylic polymer in an amount of about 3.0% by weight to about 20.0% by weight, specifically about 3.0% by weight to about 15.0% by weight, more specifically about 4.0% by weight to about 10.0% by weight, based on the dry pellet. When the amount of the acid-resistant acrylic polymer is smaller than about 3% by weight, release of the active ingredient is slow only over time, and thus it is difficult to control release of the drug at a desired site. When the amount of the acid-resistant acrylic polymer is larger than about 20% by weight, there is concern about release of a large amount of the active ingredient at once under particular pH conditions.

The two or more kinds of the insoluble diluent refer to materials that allow the granules to maintain their shape without dissolving in the body, and the insoluble diluent may be any diluent commonly used in the preparation of granules. The insoluble diluent may be selected from microcrystalline cellulose, talc, TiOz, or a combination thereof, but is not limited thereto.

The insoluble diluent may be used in an amount of about 70.0% by weight to about 95.9% by weight, specifically about 75.0% by weight to about 95.9% by weight, more specifically about 80.0% by weight to about 94.3% by weight, and in other specific range of about 70.0% by weight to about 93.0% by weight, more specifically about 75.0% by weight to about 93.0% by weight, based on the dry pellet. When the amount of the insoluble diluent is smaller than about 70.0% by weight, the pellets dissolve in the body not to maintain their shape, and accordingly, it is difficult to obtain the sustained-release capability. When the amount of the insoluble diluent is larger than about 95.5% by weight, the release amount of the drug may be lower than the desired range (about 80% or less), and accordingly, there is concern about not maintaining a target blood concentration.

The sustained-release pellets included in the pharmaceutical formulation for oral administration according to the present disclosure are required to contain two or more kinds of insoluble diluent which are different from each other in the particle size. When the insoluble diluents having different sizes are used, it is possible to control density of the pellet, and accordingly, release of the sustained-release pellets may be more easily controlled.

According to the invention, the pharmaceutical formulation for oral administration according to the present disclosure may include the tamsulosin hydrochloride-containing sustained-release pellets, each pellet containing (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, and (iv) about 70.0% by weight to about 95.9% by weight of two or more different kinds of insoluble diluent, based on the dry pellet.

In a specific embodiment, the pharmaceutical formulation for oral administration according to the present disclosure may include the tamsulosin hydrochloride-containing sustained-release pellets, each pellet containing (i) about 0.07% by weight to about 0.50% by weight of tamsulosin hydrochloride, (ii) about 1.0% by weight to about 5.0% by weight of HPMC, (iii) about 3.0% by weight to about 15.0% by weight of an acid-resistant acrylic polymer, and (iv) about 75.0% by weight to about 95.9% by weight of two or more different kinds of insoluble diluent, based on the dry pellet.

In a specific embodiment, the pharmaceutical formulation for oral administration according to the present disclosure may include the tamsulosin hydrochloride-containing sustained-release pellets, each pellet containing (i) about 0.07% by weight to about 0.50% by weight of tamsulosin hydrochloride, (ii) about 1.5% by weight to about 4.5% by weight of HPMC, (iii) about 4.0% by weight to about 10.0% by weight of an acid-resistant acrylic polymer, and (iv) about 80.0% by weight to about 94.3% by weight of two or more different kinds of insoluble diluent, based on the dry pellet.

In a specific embodiment, the pharmaceutical formulation for oral administration according to the present disclosure may include the tamsulosin hydrochloride-containing sustained-release pellets, each pellet containing (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, and (iv) about 70.0% by weight to about 93.0% by weight of two or more different kinds of insoluble diluent, based on the dry pellet.

As used herein, the term "dry pellet" refers to a sustained-release pellet that has been spheronized and dried before performing enteric coating.

The pharmaceutical formulation for oral administration according to the present disclosure may be any solid formulation including the sustained-release pellets without damage, for example, a granule formulation, a tablet formulation, a capsule formulation, etc., but is not limited thereto. In a specific embodiment, the pharmaceutical formulation may be a capsule formulation containing the sustained-release pellets.

Since the sustained-release pellets included in the pharmaceutical formulation for oral administration may contain 0.07% by weight to 0.50% by weight of tamsulosin hydrochloride, based on the dry pellet, the weight of the sustained-release granule with respect to the active ingredient is remarkably low, as compared with known tamsulosin hydrochloride capsule formulations. With regard to commercially available tamsulosin hydrochloride capsule formulations including Flomax, the weight of the granule per 0.4 mg of the active ingredient is about 330 mg, and the size of the final capsule formulation with respect to the active ingredient is large, and thus it may not be easy to swallow the capsule formulation. Further, when the capsule formulation is used as a composite formulation, the size of the capsule becomes larger than capsule size No. 1. A tamsulosin-dutasteride composite formulation (trade name: combodart, GSK) currently available abroad is a product packed in a capsule of size No. 00.

Particularly, in consideration of the fact that prostatic hypertrophy, an indication of tamsulosin hydrochloride is frequent in males over fifty and about 90% of the males over seventy suffers from prostatic hypertrophy, the size of tamsulosin hydrochloride capsule formulations is very important for patient's drug compliance.

The pharmaceutical formulation according to the present disclosure may include about 0.2 mg to about 0.4 mg of tamsulosin hydrochloride per unit formulation. In a specific embodiment, the pharmaceutical formulation is a capsule formulation including sustained-release granules, and the capsule formulation includes about 0.2 mg to about 0.4 mg of tamsulosin hydrochloride.

The hard capsule applicable to the capsule formulation of a specific embodiment of the present disclosure may be a hard capsule of any sizes that are generally used in medicine. Capsules are numbered differently depending on the sizes thereof and have different internal volumes. It is known that a capsule of size No. 00 has an internal volume of about 0.95 mL, a capsule of size No. 0 has an internal volume of about 0.68 mL, a capsule of size No. 1 has an internal volume of about 0.47 mL, a capsule of size No. 2 has an internal volume of about 0.37 mL, a capsule of size No. 3 has an internal volume of about 0.27 mL, and a capsule of size No. 4 has an internal volume about 0.20 mL (refer to the website of Suheung Capsule Co., Ltd, Korea). The size of the capsule may be smaller in consideration of drug compliance of a patient who takes the formulation of the present disclosure, but a desired unit dose of a drug may not be filled into capsules of any sizes due to mass limit of the contents filled in a capsule.

In a specific embodiment, when the pharmaceutical formulation is a capsule formulation, the capsule formulation may include sustained-release granules containing about 0.4 mg of tamsulosin hydrochloride in a capsule of size No. 3. On the contrary, tamsulosin hydrochloride capsule formulations commercially available or according to a known technology have a form in which a unit formulation containing 0.4 mg of the active ingredient is filled in a capsule of size No. 1 or larger.

In a specific embodiment, when the pharmaceutical formulation is a capsule formulation, the capsule formulation may include sustained-release granules containing about 0.2 mg of tamsulosin hydrochloride in a capsule of size No. 4, and for the preparation of a composite capsule formulation including other active ingredients together, a capsule of size No. 3 or more may be used.

In a specific embodiment, when the pharmaceutical formulation is a composite capsule formulation, the capsule formulation may be prepared as a composite capsule formulation that includes sustained-release granules containing about 0.4 mg of tamsulosin hydrochloride together with other active ingredients in a capsule of size No. 1.

In a specific embodiment, the sustained-release pellet may include (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, and (iv) about 70.0% by weight to about 95.9% by weight of two or more different kinds of insoluble diluent, based on the dry pellet, thereby greatly reducing the size of the prepared pellet and variation in the dissolution rate.

In a specific embodiment, the pharmaceutical formulation for oral administration according to the present disclosure is composed of about 65% or more of a pellet of 0.8 µm to 1.4 µm, about 15% or more of a pellet of 0.4 µm to 0.8 µm, and less than about 5% of a pellet of 0.4 µm or less, based on a diameter of spheronization, and the pellets include tamsulosin hydrochloride uniformly distributed in a carrier matrix.

The sustained-release pellet may be further coated with a sustained-release coating agent. The sustained-release coating agent may be a common enteric coating material or polymer coating material. The enteric coating material may be selected from the group consisting of, for example, hydroxypropyl methylcellulose phthalate, hydroxypropyl methylcellulose acetate succinate, polyvinyl acetate phthalate, cellulose acetate phthalate, shellac, a methacrylic acid-methyl methacrylate copolymer, a methacrylic acid-ethylacrylate copolymer, and any combination thereof, but is not limited thereto.

The polymer coating material may be selected from the group consisting of, for example, hydroxypropyl methylcellulose, methylcellulose, ethylcellulose, polyvinyl acetate, and any combination thereof, but is not limited thereto.

To secure acid resistance, the coating material may be included in an amount of about 1.5% by weight to about 15% by weight, based on the dry pellet, and to control a dissolution rate at a desired level, for example, in an amount of about 1.5% by weight, about 2% by weight, about 2.5% by weight, about 3% by weight, about 3.5% by weight, about 4.0% by weight, about 4.5% by weight, about 5.0% by weight, about 5.5% by weight, about 6.0% by weight, about 6.5% by weight, about 7.0% by weight, about 7.5% by weight, about 8.0% by weight, about 8.5% by weight, about 9.0% by weight, about 9.5% by weight, about 10% by weight, about 10.5% by weight, about 11% by weight, about 11.5% by weight, about 12% by weight, about 12.5% by weight, about 13% by weight, about 13.5% by weight, about 14% by weight, about 14.5% by weight, or about 15% by weight, or in an amount of % by weight containing about the above range, for example, in an amount of about 1.5% by weight to about 15% by weight, about 3% by weight to about 15% by weight, about 4% by weight to about 13% by weight, about 5% by weight to about 10% by weight, or about 1.5% by weight to about 3% by weight. When the coating material is used in the above range, a dissolution rate may be obtained at a desired level.

Further, when the coating material is included in an amount of 1.5% by weight to 15% by weight to control release in the stomach, it is possible to realize a tamsulosin capsule having dissolution suitable for USP monograph "Tamsulosin capsule".

In a specific embodiment, the pharmaceutical formulation for oral administration according to the present disclosure may include (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, (iv) about 70.0% by weight to about 93.0% by weight of two or more different kinds of insoluble diluent, and (v) about 1.5% by weight to about 15% by weight of an enteric coating agent, based on the dry pellet.

In another specific embodiment, the pharmaceutical formulation for oral administration according to the present disclosure may include (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, (iv) about 70.0% by weight to about 95.9% by weight of two or more different kinds of insoluble diluent, and (v) about 3% by weight to about 15% by weight of an enteric coating agent, based on the dry pellet.

In a specific embodiment, the pharmaceutical formulation for oral administration according to the present disclosure may exhibit a dissolution rate of about 30% to about 50%, specifically about 32% to about 48%, and more specifically about 35% to about 45% in 1 hour, when dissolution is performed according to a paddle method which is dissolution test II in the U.S. Pharmacopoeia (USP) under conditions of 37°C, 900 mL of pH 6.8 solution (USP artificial intestinal fluid without pancreatin) and 50 rpm.

In a specific embodiment, the pharmaceutical formulation according to the present disclosure may have sustained release capability with a nearly zero order dissolution by including the sustained-release granules. As evaluated according to a paddle method which is dissolution test II in the U.S. Pharmacopoeia (USP) including a dissolution test in 750 mL of 0.1 N HCl aqueous solution at about 37±0.5 °C and about 50 rpm for about 2 hours, continuously followed by a dissolution test at about 37±0.5 °C and about 50 rpm in an aqueous buffer solution of which pH is adjusted to pH 6.8 by adding 250 mL of a phosphate buffer, the oral pharmaceutical formulation may have a dissolution rate of tamsulosin hydrochloride of less than 10% or less than 5%, or a dissolution rate of 13% to 34% or 20% to 27% with respect to the corresponding amount of one capsule, as a result of the dissolution test for about 2 hours in 0.1 N HCl aqueous buffer solution. In addition, the oral pharmaceutical formulation may have a dissolution rate of tamsulosin hydrochloride of 80% or more with respect to the corresponding amount of one capsule in about 8 hours in the aqueous buffer solution of pH 6.8. The pharmaceutical formulation according to the present disclosure may be used for the treatment of any diseases which are known as an indication of tamsulosin hydrochloride, and for the treatment of any future diseases which will be identified as an indication of tamsulosin hydrochloride. As used herein, "treatment" may include the meaning of treatment, improvement, amelioration, and management of a disease. In a specific embodiment, the pharmaceutical formulation may be used for the treatment of benign prostatic hyperplasia, urination disturbances concomitant with prostatic hyperplasia, and acute urinary retention.

According to the invention, the pharmaceutical formulation for oral administration is prepared by a method comprising (a) mixing and extruding a mixture containing (i) tamsulosin hydrochloride, (ii) HPMC which is a non-pH-dependent polymer, (iii) an acid-resistant acrylic polymer which is a pH-dependent polymer, and (iv) two or more kinds of insoluble diluent to mold the mixture in the form of granules; and (b) spheronizing the extruded granules using a spheronizer at a rotation speed of about 600 rpm to about 800 rpm for about 10 minutes to about 45 minutes to prepare pellets.

In a specific embodiment, the preparation method of the present disclosure may include (a) mixing and extruding a mixture containing (i) about 0.07% by weight to about 0.50% by weight of tamsulosin hydrochloride, (ii) about 1.0% by weight to about 7.0% by weight of HPMC, (iii) about 3.0% by weight to about 20.0% by weight of an acid-resistant acrylic polymer, and (iv) about 70.0% by weight to about 95.9% by weight of two or more different kinds of insoluble diluent, based on the dry pellet, to mold the mixture in the form of granules; and (b) spheronizing the extruded granules using a spheronizer at a rotation speed of about 600 rpm to about 800 rpm for about 10 minutes to about 45 minutes to prepare pellets.

The above-detailed description of the pharmaceutical formulation according to one aspect of the present disclosure may also be applied to description of the method of preparing the pharmaceutical formulation.

The molding of the granules may be performed according to a method of preparing granules known in the art. In a specific embodiment, the molding of the granules may be performed by wet-grinding and extrusion-molding of the ingredients. The extrusion-molding may be performed by, for example, putting the wet ground products in an extruder.

The spheronizing may be performed by spheronizing the molded granules using a spheronizer at a rotation speed of about 600 rpm to about 800 rpm for about 10 to about 45 minutes. When the rotation speed and the time are below the ranges, sphericity may be lowered. It may also be practically difficult to set the rotation speed above the range. When the spheronizing is performed for a time over the above range, productivity may be decreased to increase generation of fine powder.

The method of preparing the pharmaceutical formulation may further include filling a capsule with the spheronized sustained-release granules to form a capsule formulation. As needed, a pharmaceutically acceptable additive may be optionally added to the sustained-release granules, which may then be filled into a hard capsule to form a capsule formulation. The pharmaceutically acceptable additive may be a plasticizer, a lubricating agent, any other adjuvants, etc.

Still another aspect of the present disclosure provides a hard capsule composite formulation containing tamsulosin hydrochloride comprising (A) tamsulosin hydrochloride-containing sustained-release pellets containing (i) tamsulosin hydrochloride, (ii) HPMC, (iii) an acid-resistant acrylic polymer, and (iv) two or more kinds of insoluble diluent; and (B) a pharmaceutical composition physically separated from the tamsulosin hydrochloride-containing sustained-release pellets, the pharmaceutical composition containing one or more different pharmaceutically active ingredients and a pharmaceutically acceptable carrier.

The above description of the pharmaceutical formulation for oral administration including the tamsulosin hydrochloride-containing sustained-release pellets according to one aspect of the present disclosure may also be applied to a detailed description of the tamsulosin hydrochloride-containing sustained-release pellet included in the tamsulosin hydrochloride-containing hard capsule composite formulation.

In a specific embodiment, the tamsulosin hydrochloride-containing hard capsule composite formulation of the present disclosure may be a tamsulosin hydrochloride-containing hard capsule composite formulation including (A) tamsulosin hydrochloride-containing sustained-release pellets each containing (i) about 0.07% by weight to about 0.50% by weight of tamsulosin hydrochloride, (ii) about 1.0% by weight to about 7.0% by weight of HPMC, (iii) about 3.0% by weight to about 25.0% by weight of an acid-resistant acrylic polymer, and (iv) about 70.0% by weight to about 95.9% by weight of two or more different kinds of insoluble diluent, based on the dry pellet; and (B) the pharmaceutical composition physically separated from the tamsulosin hydrochloride-containing sustained-release pellets, the pharmaceutical composition containing one or more different pharmaceutically active ingredients and a pharmaceutically acceptable carrier.

When the above-described pharmaceutical formulation for oral administration including the tamsulosin hydrochloride-containing sustained-release pellets according to the present disclosure includes the same unit administration dose of tamsulosin hydrochloride, the size of the unit formulation may be remarkably reduced, as compared with those of known formulations. In particular, this effect may be remarkable when applied to composite formulations.

The "including the pharmaceutical composition physically separated from the tamsulosin hydrochloride-containing sustained-release pellets, the pharmaceutical composition containing one or more different pharmaceutically active ingredients and the pharmaceutically acceptable carrier" means that they are filled into a hard capsule, each as a separate formulation, to prevent mixing of the respective active ingredients. Stability of the composition may be improved and test analysis for quality management may also be more easily performed by preventing interactions between the active ingredients by the separation.

In the tamsulosin hydrochloride-containing hard capsule composite formulation, one or more different pharmaceutically active ingredients that are included while being physically separated from the tamsulosin hydrochloride-containing sustained-release pellets may be an arbitrary drug that is known to be used in combination with tamsulosin hydrochloride. For example, to prevent or treat erectile dysfunction, benign prostatic hyperplasia, etc., dutasteride, tadalafil, finasteride, solifenacin, etc. which is known to be used in combination with tamsulosin hydrochloride may be used while being physically separated from the tamsulosin hydrochloride-containing sustained-release pellet, thereby preparing the tamsulosin hydrochloride-containing hard capsule composite formulation, but is not limited thereto.

In a specific embodiment, one or more different pharmaceutically active ingredients which may be included in the tamsulosin hydrochloride-containing hard capsule composite formulation according to the present disclosure may be dutasteride, tadalafil, finasteride, solifenacin, or a pharmaceutically acceptable salt thereof, or any mixture thereof. Specifically, for example, formulations such as a hard capsule composite formulation including the tamsulosin-containing sustained-release pellets and a dutasteride soft capsule, a polycap of the tamsulosin-containing sustained-release pellets and a tadalafil-containing tablet or pellet, the tamsulosin-containing sustained-release pellets and a finasteride-containing tablet, or a polycap of the tamsulosin-containing sustained-release pellets and a solifenacin-containing tablet or pellet, etc. may be prepared.

The pharmaceutical composition including one or more different pharmaceutically active ingredients and a pharmaceutically acceptable carrier may be prepared in the form of a pellet, a soft capsule, a tablet, etc. to be included in the tamsulosin hydrochloride-containing hard capsule composite formulation.

In the capsule formulation according to one specific embodiment of the present disclosure, capsules of size No. 0, No.1, No.2, and No.3 (including elongated capsules) may be used. Specifically, capsules of size No. 1, No.2 and No.3, and more specifically, a capsule of size No. 1 or No. 2 may be used.

In a specific embodiment of the present disclosure, the tamsulosin hydrochloride-containing sustained-release pellets, together with 0.5 mg of a commercially available Hanmi Duted soft capsule, may be filled into a hard capsule of size No. 1 to prepare a polycap composite formulation of 0.5 mg of dutasteride/0.4 mg of tamsulosin.

Further, in a specific embodiment of the present disclosure, the tamsulosin hydrochloride-containing sustained-release pellets, together with a tadalafil pellet or tablet, may be filled into a hard capsule of size No. 1 to prepare a polycap composite formulation of 5 mg of tadalafil/0.4 mg of tamsulosin.

Further, in a specific embodiment of the present disclosure, the tamsulosin hydrochloride-containing sustained-release pellets, together with a solifenacin tablet, may be filled into a hard capsule of size No. 1 to prepare a polycap composite formulation of 5 mg of solifenacin/0.2 mg of tamsulosin.

### MODE OF DISCLOSURE

### [Example]

Hereinafter, the present disclosure will be described in detail with reference to the following Examples. However, these Examples are for illustrative purposes only, and the scope of the present disclosure is not intended to be limited thereby.

### Examples 1 to 12 and Comparative Examples 1 to 10: Preparation of tamsulosin hydrochloride-containing sustained-release dry pellets

Tamsulosin hydrochloride; hydroxypropyl methylcellulose (HPMC) (METOLOSE 90SH) having a viscosity of 100,000 cps (100 Pa.s); Eudragit L30 D-55, Eudragit L100 D-55, or Eudragit L12,5; microcrystalline cellulose (MCC); and talc were put in respective amounts (unit: mg) described in Tables 1 to 4 in a high speed mixer and mixed with each other, followed by wet-grinding. The ground product was put in an extruder in which a sieve having a size of 0.8 mm was inserted, and extruded at a screw speed of 35 rpm, and a core pellet was prepared using a spheronizer at a rotation speed of 750 rpm for 25 minutes.

**[Table 1]**

| Examples 1 to 6: Preparation of dry pellets according to each prescription (1) | | | | | | |
|---|---|---|---|---|---|---|
| | Example 1 | Example2 | Example 3 | Example4 | Example5 | Examples |
| Tamsulosin HCl | 0.4 | 0.4 | 0.4 | 0.4 | 0.4 | 0.4 |
| HPMC | 3.0 | 1.5 | 2.0 | 4.0 | 8.0 | 3.0 |
| MCC | 110.0 | 110.0 | 110.0 | 110.0 | 110.0 | 110.0 |
| Talc | 11.0 | 11.0 | 5.0 | 5.0 | 11.0 | 11.0 |
| Eudragit L30 D-55 | 9.0 | 9.0 | 9.0 | 9.0 | 9.0 | 6.0 |
| Eudragit L100 D-55 | - | - | - | - | - | - |
| Eudragit L12,5 | - | - | - | - | - | - |
| Total | 133.4 | 131.9 | 126.4 | 128.4 | 138.4 | 130.4 |

**[Table 2]**

| Examples 6 to 12: Preparation of dry pellets according to each prescription (2) | | | | | | |
|---|---|---|---|---|---|---|
| | Example 7 | Example 8 | Example 9 | Example 10 | Example 11 | Example 12 |
| Tamsulosin HCl | 0.4 | 0.4 | 0.4 | 0.4 | 0.4 | 0.4 |
| HPMC | 3.0 | 6.0 | 1.0 | 6.0 | 3.0 | 3.0 |
| MCC | 110.0 | 110.0 | 82.5 | 198.0 | 110.0 | 110.0 |
| Talc | 11.0 | 22.0 | 8.3 | 19.8 | 11.0 | 11.0 |
| Eudragit L30 D-55 | 27.0 | 27.0 | 6.8 | 16.2 | - | - |
| Eudragit L100 D-55 | - | - | - | - | 9.0 | - |
| Eudragit L12,5 | - | - | - | - | - | 9.0 |
| Total | 151.4 | 165.4 | 99 | 240.4 | 133.4 | 133.4 |

**[Table 3]**

| Comparative Examples 1 to 6: Preparation of dry pellets according to each prescription (1) | | | | | | |
|---|---|---|---|---|---|---|
| | Comparative Example 1 | Comparative Example 2 | Comparative Example 3 | Comparative Example 4 | Comparative Example 5 | Comparative Example 6 |
| Tamsulosin HCl | Commercial product (Flomax 0.4 mg) | Commercial product (Combodart) | 0.4 | 0.4 | 0.4 | 0.4 |
| HPMC | | | 0.0 | 1.0 | 12.0 | 1.0 |
| MCC | | | 110.0 | 110.0 | 110.0 | 110.0 |
| Talc | | | 11.0 | 11.0 | 11.0 | 11.0 |
| Eudragit L30 D-55 | | | 13.2 | 13.2 | 13.2 | 3.0 |
| Total | About 330mg | - | 134.6 | 135.6 | 146.6 | 125.4 |

In the above Comparative Examples 1 and 2, three batches (i.e., Comparative Examples 1-1, 1-2 and 1-3 and Comparative Examples 2-1, 2-2 and 2-3) of each dry pellet were prepared.

**[Table 4]**

| Comparative Examples 7 to 10: Preparation of dry pellets according to each prescription (2) | | | | |
|---|---|---|---|---|
| | Comparative Example 7 | Comparative Example 8 | Comparative Example 9 | Comparative Example 10 |
| Tamsulosin HCl | 0.4 | 0.4 | 0.4 | 0.4 |
| HPMC | 8.0 | 8.0 | 1.0 | 1.5 |
| MCC | 110.0 | 82.5 | 198 | 110.0 |
| Talc | 11.0 | 8.25 | 19.8 | 0.0 |
| Eudragit L30 D-55 | 45.0 | 6.75 | 16.2 | 9.0 |
| Total | 174.4 | 105.9 | 235.4 | 120.9 |

### Examples 3-1 to 3-4: Enteric coating of dry pellets of Example 3 according to ratio

A coating solution including 26.7 parts by weight of a methacrylic acid-ethylacrylate copolymer (8.0 parts by weight as a solid content), 1.2 parts by weight of talc, 0.8 parts by weight of Triacetin (plasticizer), and 24.0 parts by weight of distilled water was sprayed onto the tamsulosin hydrochloride sustained-release dry pellets prepared in Example 3 using a NQ-160 fluidized bed system of DALTON (Japan) from the bottom thereof. At this time, coating was performed under conditions of an inlet temperature of 35°C to 45°C, an outlet temperature of 25°C to 35°C, a coating solution spraying rate of 7 rpm to 13 rpm, and an spraying air pressure of 500 m³/h to 1000 m³/h to obtain the tamsulosin hydrochloride sustained-release pellet coated with the sustained-release coating agent.

### Enteric coating composition: Eudragit L30 D-55

### Equipment used: Fluid bed coating machine

**[Table 5]**

| | Example 3-1 | Example 3-2 | Example 3-3 | Example 3-4 |
|---|---|---|---|---|
| Weight of dry pellet (mg/cap) | 126.4 | | | |
| Enteric coating ratio (%) | 10% | 4% | 7% | 13% |
| Final weight (mg/cap) | 139.0 | 131.5 | 135.2 | 142.8 |

### Example 13: Preparation of capsule containing dry pellet of Example 3

About 63.2 mg of the sustained-release pellets containing Example 3 in the amount corresponding to 0.2 mg of tamsulosin hydrochloride were filled into a capsule of size No. 3.

### Example 14: Preparation of capsule containing dry pellet of Example 3-1

About 69.5 mg of the sustained-release pellets containing Example 3-1 in the amount corresponding to 0.2 mg of tamsulosin hydrochloride were filled into a capsule of size No. 3.

### Experimental Example 1: Dissolution test (pH 6.8)

The dry pellets of Examples 1 to 5, Example 13, and Comparative Examples 1, 3 to 10 were subjected to a dissolution test according to the following conditions.

Dissolution was performed according to a paddle method which is dissolution test II in the U.S. Pharmacopoeia (USP) under conditions of 900 mL of pH 6.8 solution (USP artificial intestinal fluid without pancreatin) and 50 rpm, and sampling was performed at time points of 15 minutes, 30 minutes, and 60 minutes. The samples were filtered through a 0.45 µm filter, followed by analysis under the following HPLC analysis conditions.
- Column: a column in which a stainless steel tube having an inner diameter of about 4.6 mm and a length of 5 cm was packed with octadecylsilanized silica gel for liquid chromatography having a diameter of 5 µm (Kromasil Eternity-5-C18, AkzoNobel)
- Detector: Ultraviolet spectrophotometer (measured at a wavelength of 225 nm)
- Flow rate: 1.3 mL/min
- Input: 100 µl
- Column temperature: 30 °C
- Mobile phase: 8.7 mL of perchloric acid and 3.0 g of sodium hydroxide were dissolved in 1900 mL of water, and then this mixture was adjusted to pH 2.0 with sodium hydroxide, and filled with water to 2000 mL. 480 mL of acetonitrile was added to 1520 mL of the resulting solution to obtain a mobile phase.

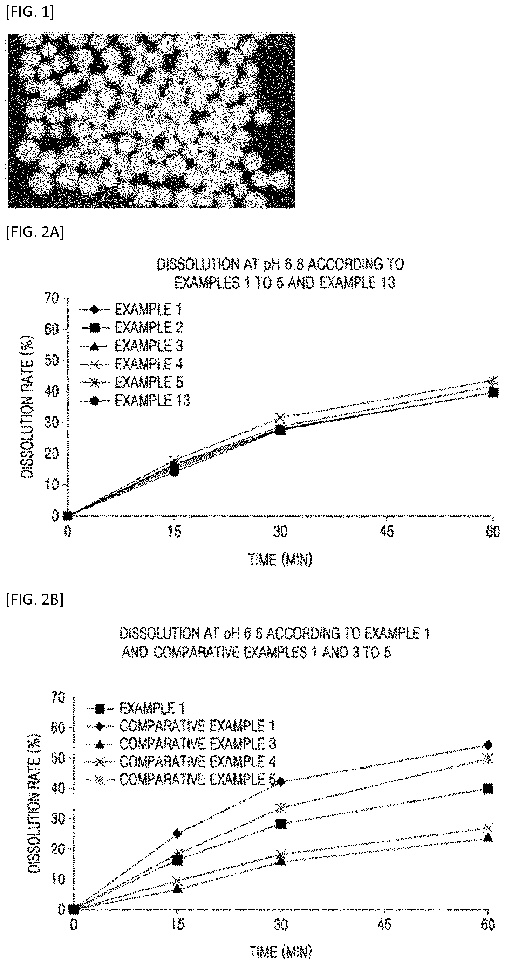

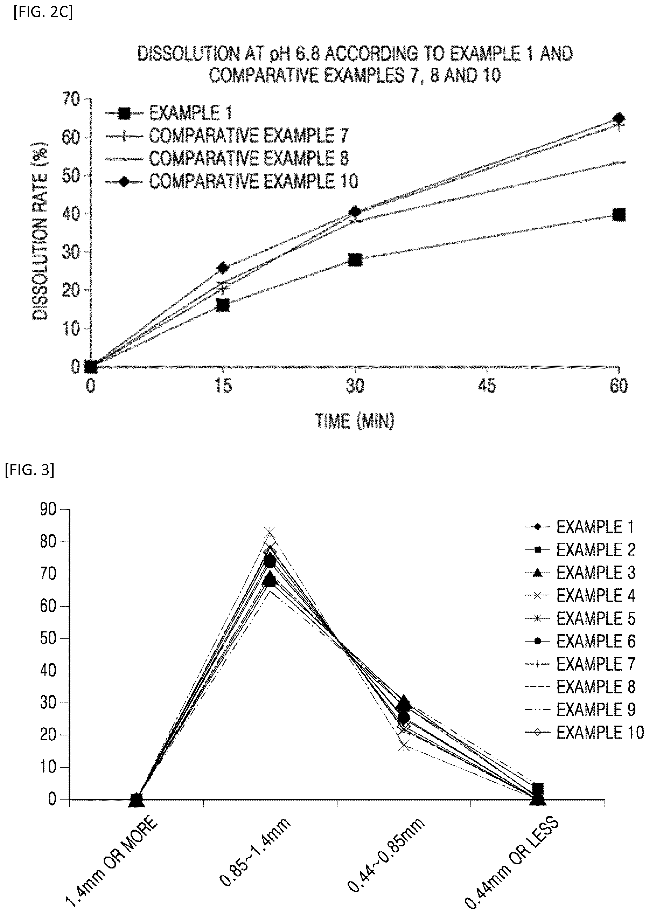

Results of measuring dissolution rates of the dry pellets of Examples 1 to 5, Example 13 and Comparative Examples 1 and 3 to 10 are summarized in the following Tables 6 and 7, and shown in FIGS. 2A to 2C.

**[Table 6]**

| Time (min) | Example 1 | Example 2 | Example 3 | Example 4 | Example 5 | Example 13 |
|---|---|---|---|---|---|---|
| 0 | 0.0 | 0.0 | 0.0 | 0.0 | 0.0 | 0.0 |
| 15 | 16.6 | 15.6 | 15.9 | 16.6 | 17.7 | 15.0 |
| 30 | 28.4 | 27.5 | 28.0 | 28.7 | 31.4 | 27.4 |
| 60 | 40.1 | 39.5 | 40.0 | 41.6 | 43.3 | 39.5 |

**[Table 7]**

| Time (min) | Comparat ive Example 1 | Comparat ive Example 3 | Comparat ive Example 4 | Comparat ive Example 5 | Comparat ive Example 6 | Comparat ive Example 7 | Comparat ive Example 8 | Comparat ive Example 9 | Comparat ive Example 10 |
|---|---|---|---|---|---|---|---|---|---|
| 0 | 0.0 | 0.0 | 0.0 | 0.0 | 0.0 | 0.0 | 0.0 | 0.0 | 0.0 |
| 15 | 25.4 | 7.3 | 9.5 | 18.4 | 11.4 | 20.7 | 22.4 | 7.0 | 26.2 |
| 30 | 42.6 | 16.6 | 18.4 | 33.5 | 20.5 | 40.2 | 38.2 | 14.9 | 40.6 |
| 60 | 54.6 | 24.3 | 27.1 | 50.1 | 27.8 | 63.4 | 53.6 | 21.4 | 64.9 |

As shown in FIGS. 2A to 2C, the dry pellets of Examples 1 to 5 showed similar dissolution patterns, and Example 13 packed with the corresponding amount of 0.2 mg also showed a similar dissolution pattern to Example 3. However, Comparative Examples 1 and 3 to 10 in which the content of the added excipient was out of the range of the present disclosure showed different dissolution rates from each other, and it was difficult to obtain dissolution rates at target levels, suggesting that it is difficult to control release behaviors in the body.

### Experimental Example 2: Pellet size test

### The tamsulosin hydrochloride sustained-release pellets prepared in Examples 1 to 12 and Comparative Examples 1 to 10 were filtered through a sieve of size No. 35 (market grade, sieve size: 0.44 mm), a sieve of size No. 20 (market grade, sieve size: 0.85 mm) and a sieve of size No. 14 (market grade, sieve size: 1.4 mm), and a remaining portion on the sieve of size No. 14, a remaining portion on the sieve of size No. 20, and a remaining portion on the sieve of size No. 35, and a portion passed through the sieve of size No. 35 were collected, and ratios (unit:% by weight) of particles having the pellet sizes of 1.4 mm or more, 0.85 mm to 1.4 mm, 0.44 mm to 0.85 mm, and 0.44 mm or less were determined, and the results are shown in the following Tables 8 to 10.

**[Table 8]**

| Particle size of sustained-release pellet | Exam ple 1 | Exam ple 2 | Exam ple 3 | Exam ple 4 | Exam ple 5 | Exam ple 6 | Exam ple 7 | Exam ple 8 | Exam ple 9 | Exam ple 10 | Exam ple 11 | Exam ple 12 |
|---|---|---|---|---|---|---|---|---|---|---|---|---|
| 1.4 mm or more | 0.0 | 0.0 | 0.0 | 0.0 | 0.2 | 0.0 | 0.0 | 0.1 | 0.0 | 0.1 | 0.0 | 0.0 |
| 0.85 mm to 1.4 mm | 75.0 | 67.9 | 69.5 | 78.3 | 83.2 | 74.2 | 70.6 | 78.7 | 65.3 | 77.2 | 74.6 | 76.3 |
| 0.44 mm to 0.85 mm | 24.7 | 28.9 | 30.2 | 21.5 | 16.6 | 25.5 | 28.9 | 21.0 | 30.6 | 22.4 | 25.1 | 23.4 |
| 0.44 mm or less | 0.3 | 3.2 | 0.3 | 0.2 | 0.0 | 0.3 | 0.5 | 0.2 | 4.1 | 0.3 | 0.3 | 0.3 |
| | 100 | 100 | 100 | 100 | 100 | 100 | 100 | 100 | 100 | 100 | 100 | 100 |

**[Table 9]**

| Particle size of sustained-release pellet | Comparative Example 1 | | | Comparative Example 2 | | |
|---|---|---|---|---|---|---|
| | Comparativ e Example 1-1 | Comparativ e Example 1-2 | Comparativ e Example 1-3 | Comparativ e Example 2-1 | Comparativ e Example 2-2 | Comparativ e Example 2-3 |
| 1.4 mm or more | 0.0 | 0.0 | 0.0 | 1.2 | 4.1 | 5.4 |
| 0.85 mm to 1.4 mm | 0.4 | 0.0 | 0.0 | 43.7 | 38.4 | 49.5 |
| 0.44 mm to 0.85 mm | 26.1 | 67.5 | 54.3 | 52.7 | 48.7 | 38.4 |
| 0.44 mm or less | 73.5 | 32.5 | 45.7 | 2.4 | 8.8 | 6.7 |
| | 100 | 100 | 100 | 100 | 100 | 100 |

**[Table 10]**

| Particle size of sustained-release pellet | Compar ative Example 3 | Compar ative Example 4 | Compar ative Example 5 | Compar ative Example 6 | Compar ative Example 7 | Compar ative Example 8 | Compar ative Example 9 | Comparat ive Example 10 |
|---|---|---|---|---|---|---|---|---|
| 1.4 mm or more | 40.2 | 37.8 | 15.7 | 0.0 | 1.4 | 12.6 | 30.6 | 0.0 |
| 0.85 mm to 1.4 mm | 11.8 | 12.5 | 70.3 | 79.2 | 63.0 | 75.3 | 33.9 | 75.6 |
| 0.44 mm to 0.85 mm | 23.4 | 31.2 | 14.0 | 20.1 | 33.0 | 12.1 | 25.3 | 24.3 |
| 0.44 mm or less | 24.6 | 18.5 | 0.0 | 0.7 | 2.6 | 0.0 | 10.2 | 0.1 |
| | 100 | 100 | 100 | 100 | 100 | 100 | 100 | 100 |

Part of the results is shown in FIG. 3. As shown in FIG. 3, the sustained-release pellets of Examples 1 to 10 showed uniform particle size ranges whereas Comparative Examples were expected to show a large dissolution variation between batches or within batches because a large amount of pellets of 1.4 mm or more or 0.44 mm or less existed.

### Experimental Example 3: Dissolution test (pH 6.8)-Test of reproducibility between batches

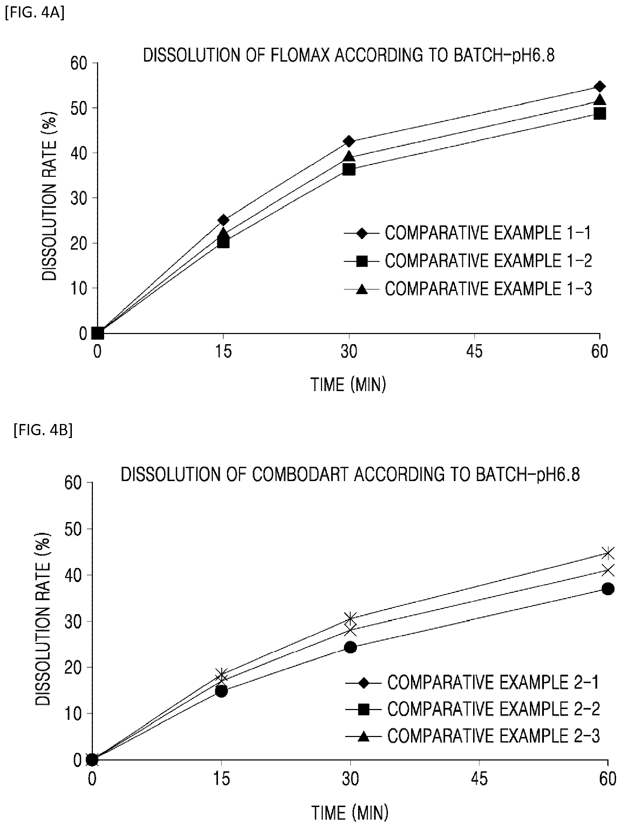

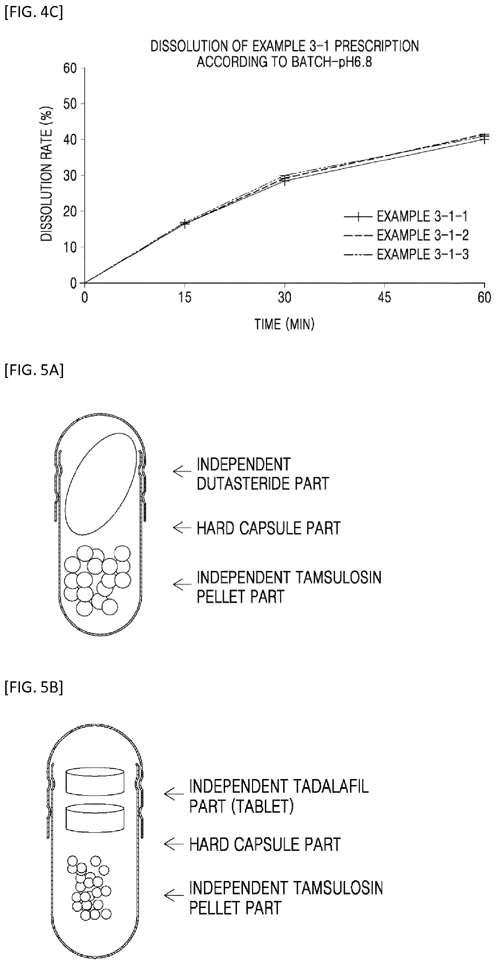

The pellets prepared in Comparative Examples 1 and 2 and Example 3-1, each divided into three batches, were subjected to the dissolution test according to the dissolution test method described in Experimental Example 1, and results are summarized in the following Tables 11 and 12 and FIGS. 4A to 4C.

**[Table 11]**

| Time (min) | Comparative Example 1 | | | Comparative Example 2 | | | Example 3-1 | | |
|---|---|---|---|---|---|---|---|---|---|
| | Compar ative Example 1-1 | Compa rative Exampl e 1-2 | Compar ative Example 1-3 | Compar ative Example 2-1 | Compar ative Example 2-2 | Compar ative Example 2-3 | Example 3-1-1 | Example 3-1-2 | Example 3-1-3 |
| 0 | 0.0 | 0.0 | 0.0 | 0.0 | 0.0 | 0.0 | 0.0 | 0.0 | 0.0 |
| 15 | 25.4 | 20.3 | 22.4 | 17.0 | 18.3 | 15.2 | 16.6 | 16.7 | 16.4 |
| 30 | 42.6 | 36.6 | 39.4 | 28.4 | 30.5 | 24.6 | 28.4 | 29.7 | 29.0 |
| 60 | 54.6 | 48.7 | 52.1 | 41.2 | 44.8 | 37.6 | 40.1 | 41.0 | 41.3 |

**[Table 12]**

| Time (min) | Comparative Example 1 (mean ± deviation) | Comparative Example 2 (mean ± deviation) | Example 3-1 (mean ± deviation) |
|---|---|---|---|
| 0 | 0.0 | 0.0 | 0.0 |
| 15 | 22.7±2.6 | 16.8±1.6 | 16.6±0.2 |
| 30 | 39.5±3.0 | 27.8±3.0 | 29.0±0.7 |
| 60 | 51.8±3.0 | 41.2±3.6 | 40.8±0.6 |

As shown in Tables 11 and 12 and FIGS. 4A to 4C, Comparative Examples 1 and 2 which are commercial products showed a difference in the dissolution rate between batches whereas Example 3-1 according to the present disclosure showed excellent reproducibility between batches, indicating that products showing identical dissolution patterns may be consistently produced, and therefore, it is expected to increase therapeutic effects on patients.

### Experimental Example 4: Dissolution test (0.1 N HCl 750mL→ 250 mL further added, pH 6.8, 1000 mL)

Enteric coated sustained-release pellets prepared in Example 3-1 to Example 3-4 and Example 14 were subjected to a dissolution test according to the following method.

### <Tamsulosin dissolution conditions>

A dissolution test was performed according to the Paddle method of the USP dissolution test II with a sinker at 50 rpm using 750 mL of an acidic solution for initial 120 minutes. Then, 250 mL of a buffer test solution was further added, and the dissolution teste was performed for 480 minutes. Samples for the dissolution test were collected in the amount of 10 mL at the initial stage, and after 120 minutes, 180 minutes, 240 minutes, 360 minutes, and 480 minutes, respectively. The collected samples were analyzed under the following HPLC analysis conditions, and tamsulosin hydrochloride dissolution rates thereof (unit: %) were calculated.
- Eluent: acidic solution - 0.1N hydrochloric acid solution
- Buffer test solution - 0.2 M Sodium phosphate tribasic (12-hydrate), pH 6.8

**[Table 13]**

| Time(h) | Example 3-1 | Example 3-2 | Example 3-3 | Example 3-4 | Example 14 |
|---|---|---|---|---|---|
| 0 | 0.0 | 0.0 | 0.0 | 0.0 | 0.0 |
| 2 | 1.3 | 3.5 | 2.1 | 1.1 | 1.2 |
| 3 | 59.3 | 61.2 | 60.1 | 58.6 | 60.1 |
| 8 | 97.5 | 97.0 | 97.7 | 97.3 | 97.9 |

Examples 3-1 to 3-4 were prepared by different enteric coating of 10% by weight, 4% by weight, 7% by weight, and 13% by weight with respect to the sustained-release pellet of the same prescription, and the sustained-release pellets of Examples 3-1 to 3-4 showed dissolution rates of 5% or less in 2 hours, and also showed dissolution rates of desired levels in 3 hours and 8 hours, indicating that there was no difference in the dissolution rate according to enteric coating levels when enteric coating of 4% by weight to 13% by weight was performed.

Further, Example 14 packed with the amount corresponding to 0.2 mg also showed a similar dissolution pattern to Example 3-1, indicating that there was no difference in the dissolution rate according to the dose.

### Examples 15 to 17: Production of polycap composite formulations

The tamsulosin hydrochloride-containing sustained-release pellet prepared in Example 3-1 and 0.5 mg of a commercially available Hanmi Duted soft capsule were filled into a hard capsule of size No. 1 to prepare a polycap composite formulation containing 0.5 mg of dutasteride/0.4 mg of tamsulosin (Example 15).

Further, the tamsulosin hydrochloride-containing sustained-release pellet prepared in Example 3-1 and a tadalafil pellet or tablet were filled into a hard capsule of size No. 1 to prepare a polycap composite formulation containing 5 mg of tadalafil/0.4 mg of tamsulosin (Examples 16-1 and 16-2).

Further, the tamsulosin hydrochloride-containing sustained-release pellet prepared in Example 3-1 and a solifenacin tablet were filled into a hard capsule of size No. 1 to prepare a polycap composite formulation containing 5 mg of solifenacin/0.2 mg of tamsulosin (Example 17). Further, the two solifenacin tablets were filled to prepare a polycap composite formulation containing 10 mg of solifenacin/0.2 mg of tamsulosin. The solifenacin tablet was prepared according to the prescription and process of Besigum tablet commercially available in Korea, and the tablet diameter was changed to 6.0 mm to be filled in a capsule.

**[Table 14]**

| | Example 15 | Example 16-1 | Example 16-2 | Example 17 |
|---|---|---|---|---|
| Dutasteride-containing soft capsule | 120 mg | - | - | - |
| Tadalafil pellet | - | 100 mg | - | - |
| Tadalafil tablet | - | - | 100 mg | - |
| Solifenacin tablet | - | - | - | 77 mg |
| Tamsulosin pellet (Example 3-1) | 139.0 mg | 139.0 mg | 139.0 mg | 69.5 mg |
| Hard capsule of size No. 1 | 77 mg | 77 mg | 77 mg | 77 mg |

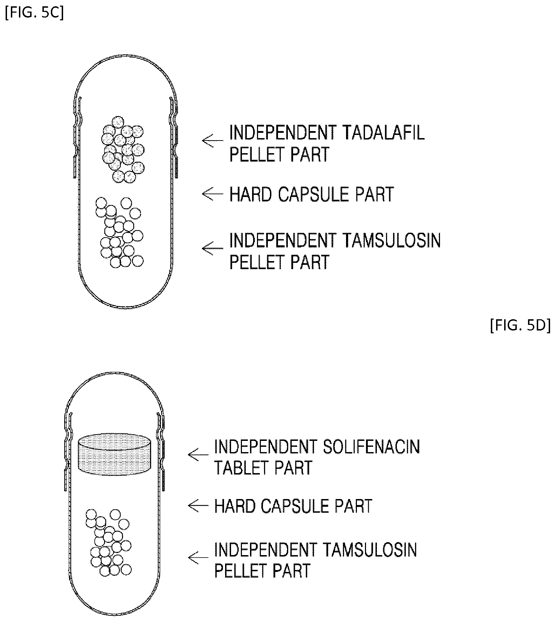

Shapes of the above prepared composite formulations are shown in FIG. 5. The above composite formulations include 0.4 mg of tamsulosin hydrochloride together with 0.5 mg of dutasteride or 5 mg of tadalafil, or include 0.2 mg of tamsulosin hydrochloride together with 5 mg or 10 mg of solifenacin, but the total weight thereof is merely 300 mg, and thus the finally prepared composite formulations have a small size, which is advantageous in that they are easy to swallow.

The present disclosure has been described with reference to preferred embodiments thereof. It will be understood by those skilled in the art to which the present disclosure pertains that the present disclosure may be implemented in a different specific form without changing the essential characteristics thereof. Therefore, it should be understood that the above embodiments are not limitative, but illustrative in all aspects. The scope of the present disclosure is defined by the appended claims rather than by the foregoing description.

## Claims

1. A pharmaceutical formulation for oral administration comprising tamsulosin hydrochloride-containing sustained-release pellets, each pellet comprising (i) tamsulosin hydrochloride, (ii) hydroxypropyl methylcellulose (HPMC), (iii) an acid-resistant acrylic polymer, and (iv) two or more kinds of insoluble diluent,
wherein the pharmaceutical formulation for oral administration is prepared by a method comprising:
(a) mixing and extruding a mixture comprising (i) tamsulosin hydrochloride, (ii) hydroxypropyl methylcellulose (HPMC), (iii) an acid-resistant acrylic polymer, and (iv) two or more kinds of insoluble diluent to mold the mixture in the form of granules; and
(b) spheronizing the extruded granules using a spheronizer at a rotation speed of about 600 rpm to about 800 rpm for about 10 minutes to about 45 minutes to prepare pellets,
wherein the sustained-release pellets each comprise (i) 0.07% by weight to 0.50% by weight of tamsulosin hydrochloride, (ii) 1.0% by weight to 7.0% by weight of hydroxypropyl methylcellulose (HPMC), (iii) 3.0% by weight to 20.0% by weight of an acid-resistant acrylic polymer, and (iv) 70.0% by weight to 95.9% by weight of two or more kinds of insoluble diluent, based on a dry pellet, and
wherein the hydroxypropyl methylcellulose (HPMC) has a viscosity of 10000 cPs to 100000 cPs (10 Pa.s to 100 Pa.s).

2. The pharmaceutical formulation for oral administration of claim 1, wherein the acid-resistant acrylic polymer is selected from the group consisting of Eudragit L30 D-55, Eudragit L100 D-5, Eudragit L12,5, and any combination thereof.

3. The pharmaceutical formulation for oral administration of claim 1, wherein the two or more kinds of insoluble diluent are selected from the group consisting of microcrystalline cellulose, talc, titanium dioxide, and any combination thereof.

4. The pharmaceutical formulation for oral administration of claim 1, wherein the sustained-release pellets are further coated with an enteric coating agent.

5. The pharmaceutical formulation for oral administration of claim 4, wherein the sustained-release pellets each comprise (i) 0.07% by weight to 0.50% by weight of tamsulosin hydrochloride, (ii) 1.0% by weight to 7.0% by weight of hydroxypropyl methylcellulose (HPMC), (iii) 3.0% by weight to 20.0% by weight of an acid-resistant acrylic polymer, (iv) 70.0% by weight to 93.0% by weight of two or more kinds of insoluble diluent, and (v) 1.5% by weight to 15% by weight of an enteric coating agent, based on a dry pellet.

6. The pharmaceutical formulation for oral administration of claim 1, wherein the sustained-release pellets are composed of 65% or more of a pellet of 0.8 µm to 1.4 µm, 15% or more of a pellet of 0.4 µm to 0.8 µm, and less than 5% of a pellet of 0.4 µm or less, based on a diameter of spheronization.

7. The pharmaceutical formulation for oral administration of claim 1, wherein the tamsulosin hydrochloride-comprising sustained-release pellets show a dissolution rate of 25% to 55% in 1 hour, as tested according to a paddle method which is dissolution test II in the U.S. Pharmacopoeia (USP) under conditions of 50 rpm, 900 mL of a pH 6.8 solution, and 37 °C.

8. The pharmaceutical formulation for oral administration of claim 1, wherein the pharmaceutical formulation is a capsule formulation comprising the sustained-release pellets.

9. The pharmaceutical formulation for oral administration of claim 1, comprising 0.2 mg or 0.4 mg of tamsulosin hydrochloride per unit formulation.

10. The pharmaceutical formulation for oral administration of claim 1 for use in the treatment or prevention of benign prostatic hyperplasia.

11. A method of preparing the pharmaceutical formulation for oral administration of claim 1, the method comprising:
(a) mixing and extruding a mixture comprising (i) tamsulosin hydrochloride, (ii) hydroxypropyl methylcellulose (HPMC), (iii) an acid-resistant acrylic polymer, and (iv) two or more kinds of insoluble diluent to mold the mixture in the form of granules; and
(b) spheronizing the extruded granules using a spheronizer at a rotation speed of about 600 rpm to about 800 rpm for about 10 minutes to about 45 minutes to prepare pellets.

12. A hard capsule composite formulation containing tamsulosin hydrochloride comprising (A) a first pharmaceutical composition and (B) a second pharmaceutical composition physically separated from the first pharmaceutical composition (A):
wherein the first pharmaceutical composition (A) is the pharmaceutical formulation of claim 1,
wherein the second pharmaceutical composition (B) comprises one or more pharmaceutically active ingredients different from tamsulosin hydrochloride and a pharmaceutically acceptable carrier.

13. The tamsulosin hydrochloride-containing hard capsule composite formulation of claim 12, wherein the one or more pharmaceutically active ingredients are selected from the group consisting of dutasteride, tadalafil, finasteride, solifenacin, a pharmaceutically acceptable salt thereof, and any combination thereof.

## Patentansprüche

1. Pharmazeutische Formulierung zur oralen Verabreichung, umfassend Tamsulosin-Hydrochlorid enthaltende Pellets mit anhaltender Freisetzung, wobei jedes Pellet (i) Tamsulosin-Hydrochlorid, (ii) Hydroxypropylmethylcellulose (HPMC), (iii) ein säureresistentes Acrylpolymer und (iv) zwei oder mehr Arten unlöslichen Verdünnungsmittels umfasst,
wobei die pharmazeutische Formulierung zur oralen Verabreichung durch ein Verfahren hergestellt wird, welches umfasst:
(a) Mischen und Extrudieren eines (i) Tamsulosin-Hydrochlorid, (ii) Hydroxypropylmethylcellulose (HPMC), (iii) ein säureresistentes Acrylpolymer und (iv) zwei oder mehr Arten unlöslichen Verdünnungsmittels umfassenden Gemischs, um das Gemisch zu Körnchen zu formen; und
(b) Runden der extrudierten Körnchen mittels eines Sphäronizers mit einer Drehzahl von etwa 600 rpm bis etwa 800 rpm für etwa 10 Minuten bis etwa 45 Minuten, um Pellets herzustellen,
wobei die Pellets mit anhaltender Freisetzung jeweils (i) 0,07 Gew.-% bis 0,50 Gew.-% Tamsulosin-Hydrochlorid, (ii) 1,0 Gew.-% bis 7,0 Gew.-% Hydroxypropylmethylcellulose (HPMC), (iii) 3,0 Gew.-% bis 20,0 Gew.-% eines säureresistenten Acrylpolymers und (iv) 70,0 Gew.-% bis 95,9 Gew.-% von zwei oder mehr Arten unlöslichen Verdünnungsmittels, bezogen auf ein trockenes Pellet, umfassen, und
wobei die Hydroxypropylmethylcellulose (HPMC) eine Viskosität von 10000 cPs bis 100000 cPs (10 Pa.s bis 100 Pa.s) aufweist.

2. Pharmazeutische Formulierung zur oralen Verabreichung nach Anspruch 1, wobei das säureresistente Acrylpolymer aus der Gruppe ausgewählt ist, die aus Eudragit L30 D-55, Eudragit L100 D-5, Eudragit L12,5 und jeder beliebigen Kombination davon besteht.

3. Pharmazeutische Formulierung zur oralen Verabreichung nach Anspruch 1, wobei die zwei oder mehr Arten unlöslichen Verdünnungsmittels aus der Gruppe ausgewählt sind, die aus mikrokristalliner Cellulose, Talk, Titandioxid und jeder beliebigen Kombination davon besteht.

4. Pharmazeutische Formulierung zur oralen Verabreichung nach Anspruch 1, wobei die Pellets mit anhaltender Freisetzung ferner mit einem magensaftresistenten Mittel überzogen sind.

5. Pharmazeutische Formulierung zur oralen Verabreichung nach Anspruch 4, wobei die Pellets mit anhaltender Freisetzung jeweils (i) 0,07 Gew.-% bis 0,50 Gew.-% Tamsulosin-Hydrochlorid, (ii) 1,0 Gew.-% bis 7,0 Gew.-% Hydroxypropylmethylcellulose (HPMC), (iii) 3,0 Gew.-% bis 20,0 Gew.-% eines säureresistenten Acrylpolymers, (iv) 70,0 Gew.-% bis 93,0 Gew.-% von zwei oder mehr Arten unlöslichen Verdünnungsmittels und (v) 1,5 Gew.-% bis 15 Gew.-% eines magensaftresistenten Mittels, bezogen auf ein trockenes Pellet, umfassen.

6. Pharmazeutische Formulierung zur oralen Verabreichung nach Anspruch 1, wobei sich die Pellets mit anhaltender Freisetzung aus 65% oder mehr von einem Pellet mit 0,8 µm bis 1,4 µm, 15% oder mehr von einem Pellet mit 0,4 µm bis 0,8 µm, und weniger als 5% von einem Pellet mit 0,4 µm oder weniger, bezogen auf einen Sphäronisierungsdurchmesser, zusammensetzen.

7. Pharmazeutische Formulierung zur oralen Verabreichung nach Anspruch 1, wobei die Tamsulosin-Hydrochlorid umfassenden Pellets mit anhaltender Freisetzung eine Auflösungsrate von 25% bis 55% in 1 Stunde zeigen, gemäß Prüfung nach einem Blattrührer- (Paddle-) Verfahren, welches Auflösungsprüfung II im US-amerikanischen Arzneibuch (U.S. Pharmacopoeia (USP)) ist, unter den Bedingungen 50 rpm, 900 mL einer pH-6,8-Lösung und 37 °C.

8. Pharmazeutische Formulierung zur oralen Verabreichung nach Anspruch 1, wobei die pharmazeutische Formulierung eine Kapselformulierung ist, welche die Pellets mit anhaltender Freisetzung umfasst.

9. Pharmazeutische Formulierung zur oralen Verabreichung nach Anspruch 1, welche 0,2 mg oder 0,4 mg Tamsulosin-Hydrochlorid pro Formulierungseinheit umfasst.

10. Pharmazeutische Formulierung zur oralen Verabreichung nach Anspruch 1, zur Verwendung bei der Behandlung oder Verhütung benigner Prostatahyperplasie.

11. Verfahren zur Herstellung der pharmazeutischen Formulierung zur oralen Verabreichung nach Anspruch 1, wobei das Verfahren umfasst:
(a) Mischen und Extrudieren eines (i) Tamsulosin-Hydrochlorid, (ii) Hydroxypropylmethylcellulose (HPMC), (iii) ein säureresistentes Acrylpolymer und (iv) zwei oder mehr Arten unlöslichen Verdünnungsmittels umfassenden Gemischs, um das Gemisch zu Körnchen zu formen; und
(b) Runden der extrudierten Körnchen mittels eines Sphäronizers mit einer Drehzahl von etwa 600 rpm bis etwa 800 rpm für etwa 10 Minuten bis etwa 45 Minuten, um Pellets herzustellen.

12. Tamsulosin-Hydrochlorid enthaltende Hartkapselverbundformulierung, umfassend (A) eine erste pharmazeutische Zusammensetzung und (B) eine zweite pharmazeutische Zusammensetzung, die von der ersten pharmazeutischen Zusammensetzung (A) körperlich getrennt ist:
wobei die erste pharmazeutische Zusammensetzung (A) die pharmazeutische Formulierung nach Anspruch 1 ist,
wobei die zweite pharmazeutische Zusammensetzung (B) einen oder mehrere pharmazeutisch aktive Inhaltsstoffe, anders als Tamsulosin-Hydrochlorid, und einen pharmazeutisch verträglichen Träger umfasst.

13. Tamsulosin-Hydrochlorid enthaltende Hartkapselverbundformulierung nach Anspruch 12, wobei der eine oder die mehreren pharmazeutisch aktiven Inhaltsstoffe aus der Gruppe ausgewählt sind, die aus Dutasterid, Tadalafil, Finasterid, Solifenacin, einem pharmazeutisch verträglichen Salz davon und jeder beliebigen Kombination davon besteht.

## Revendications

1. Formulation pharmaceutique pour administration par voie orale comprenant des pastilles à libération prolongée contenant du chlorhydrate de tamsulosine, chaque pastille comprenant (i) un chlorhydrate de tamsulosine, (ii) de l'hydroxypropyl-méthylcellulose (HPMC), (iii) un polymère acrylique résistant à l'acide, et (iv) deux types de diluant insoluble ou plus,
dans lequel la formulation pharmaceutique pour administration par voie orale est préparée par un procédé comprenant :
(a) le mélange et l'extrusion d'un mélange comprenant (i) un chlorhydrate de tamsulosine, (ii) de l'hydroxypropyl-méthylcellulose (HPMC), (iii) un polymère acrylique résistant à l'acide, et (iv) deux types de diluant insoluble ou plus pour mouler le mélange sous la forme de granules ; et
(b) la sphéronisation des granules extrudés en utilisant une machine de sphéronisation à une vitesse de rotation d'environ 600 trs/mn à environ 800 trs/mn pendant environ 10 minutes à environ 45 minutes pour préparer des pastilles,
dans laquelle les pastilles à libération prolongée comprennent chacune (i) 0,07 % en poids à 0,50 % en poids de chlorhydrate de tamsulosine, (ii) 1,0 % en poids à 7,0 % en poids d'hydroxypropyl-méthylcellulose (HPMC), (iii) 3,0 % en poids à 20,0 % en poids d'un polymère acrylique résistant à l'acide, et (iv) 70,0 % en poids à 95,9 % en poids de deux types ou plusieurs de diluant insoluble, sur la base de la pastille sèche, et
dans laquelle l'hydroxypropyl-méthylcellulose (HPMC) présente une viscosité de 10000 cPs à 100000 cPs (10 Pa.s à 100 Pa.s).

2. Formulation pharmaceutique pour administration par voie orale selon la revendication 1, dans laquelle le polymère acrylique résistant à l'acide est sélectionné dans le groupe constitué de l'Eudragit L30 D-55, de l'Eudragit L100 D-5, de l'Eudragit L12,5, et de toute combinaison de ceux-ci.

3. Formulation pharmaceutique pour administration par voie orale selon la revendication 1, dans laquelle les deux types ou plus de diluant insoluble sont sélectionnés dans le groupe constitué de cellulose microcristalline, de talc, de dioxyde de titane, et d'une combinaison quelconque de ceux-ci.

4. Formulation pharmaceutique pour administration par voie orale selon la revendication 1, dans laquelle les pastilles à libération prolongée sont en outre revêtues d'un agent d'enrobage entérique.

5. Formulation pharmaceutique pour administration par voie orale selon la revendication 4, dans laquelle les pastilles à libération prolongée comprennent chacune (i) 0,07 % en poids à 0,50 % en poids de chlorhydrate de tamsulosine, (ii) 1,0 % en poids à 7,0 % en poids d'hydroxypropyl-méthylcellulose (HPMC), (iii) 3,0 % en poids à 20,0 % en poids d'un polymère acrylique résistant à l'acide, et (iv) 70,0 % en poids à 93,0 % en poids de deux types ou plus de diluant insoluble, et (v) 1,5 % en poids à 15 % en poids d'un agent d'enrobage entérique, sur la base d'une pastille sèche.

6. Formulation pharmaceutique pour administration par voie orale selon la revendication 1, dans laquelle les pastilles à libération prolongée sont composées de 65 % ou plus d'une pastille de 0,8 µm à 1,4 µm, 15 % ou plus d'une pastille de 0,4 µm à 0,8 µm, et moins de 5 % d'une pastille de 0,4 µm ou moins, sur la base d'un diamètre de sphéronisation.

7. Formulation pharmaceutique pour administration par voie orale selon la revendication 1, dans laquelle les pastilles à libération prolongée comprenant du chlorhydrate de tamsulosine présentent une vitesse de dissolution de 25 % à 55 % en 1 heure, comme testé selon un procédé d'agitation paddle qui est un test de dissolution II dans la Pharmacopée américaine (USP) dans des conditions de 50 trs/mn, 900 mL d'une solution à un pH de 6,8, et à 37°C.

8. Formulation pharmaceutique pour administration par voie orale selon la revendication 1, dans laquelle la formulation pharmaceutique est une formulation de gélule comprenant les pastilles à libération prolongée.

9. Formulation pharmaceutique pour administration par voie orale selon la revendication 1, comprenant 0,2 mg ou 0,4 mg de chlorhydrate de tamsulosine par formulation unitaire.

10. Formulation pharmaceutique pour administration par voie orale selon la revendication 1, pour utilisation dans le traitement ou la prévention d'une hyperplasie prostatique bénigne.

11. Procédé de préparation de la formulation pharmaceutique pour une administration par voie orale selon la revendication 1, le procédé comprenant :
(a) le mélange et l'extrusion d'un mélange comprenant (i) du chlorhydrate de tamsulosine, (ii) de l'hydroxypropyl-méthylcellulose (HPMC), (iii) un polymère acrylique résistant à l'acide, et (iv) deux types de diluant insoluble ou plus pour mouler le mélange sous la forme de granules ; et
(b) la sphéronisation des granules extrudés en utilisant une machine de sphéronisation à une vitesse de rotation d'environ 600 trs/mn à environ 800 trs/mn pendant environ 10 minutes à environ 45 minutes pour préparer des pastilles.

12. Formulation composite de gélule dure contenant du chlorhydrate de tamsulosine comprenant (A) une première composition pharmaceutique et (B) une seconde composition pharmaceutique physiquement séparée de la première composition pharmaceutique (A) :
dans laquelle la première composition pharmaceutique (A) est la formulation pharmaceutique selon la revendication 1,
dans laquelle la seconde composition pharmaceutique (B) comprend un ou plusieurs ingrédients pharmaceutiquement actifs différents du chlorhydrate de tamsulosine et d'un excipient pharmaceutiquement acceptable.

13. Formulation composite de gélule dure contenant du chlorhydrate de tamsulosine selon la revendication 12, dans laquelle le ou les ingrédients pharmaceutiquement actifs sont sélectionnés dans le groupe constitué de dutastéride, tadalafil, finastéride, solifénacine, un sel pharmaceutiquement acceptable de ceux-ci et toute combinaison de ceux-ci.
